# EUROPÄISCHE PATENTANMELDUNG

(11) **EP 1 917 943 A2**
(43) Veröffentlichungstag der Anmeldung: **07.05.2008**
(21) Anmeldenummer: 07021501.7
(22) Anmeldetag: 05.11.2007
(51) Int. Cl.: A61F 13/34

(54) **Tampon**

(30) Priorität: 06.11.2006 DE 102006052206; 06.11.2006 DE 202006016949 U
(71) Anmelder: Fischer, Ruth-Maria, 77855 Achern (DE)
(72) Erfinder: Fischer, Ruth-Maria, 77855 Achern (DE)
(74) Vertreter: Vossius & Partner

(57) **Zusammenfassung**

Die vorliegende Erfindung betrifft ein Tampon insbesondere für die Frauenhygiene. Das erfindungsgemäße Tampon weist einen länglichen Tamponkörper auf, der ein Einführende, einen Mittelabschnitt und ein dem Einführende gegenüberliegendes Rückholende hat. Darüber hinaus weist das Tampon eine Rückholeinrichtung mit einem mit dem Tamponkörper verbundenen, flexiblen Zugelement, das sich vom Tamponkörper weg erstreckt, und einen Griffabschnitt auf. Der Griffabschnitt stellt bezüglich des Zugelements eine erhebliche Querschnittserweiterung bereit.

## Beschreibung

Die vorliegende Erfindung betrifft ein Tampon, insbesondere für die Frauenhygiene.

Derartige Tampons weisen üblicherweise einen länglichen Tamponkörper mit einem Einführende, einem Mittelabschnitt und einem dem Einführende gegenüberliegenden Rückholende auf. Zum Entfernen des Tampons erstreckt sich vom Rückholende ein Zugelement, das üblicherweise in Form eines Bandes oder einer Schnur ausgebildet ist. Das Band bzw. die Schnur kann in Form einer Schlaufe ausgebildet sein. Derartige Tampons sind beispielsweise in der DE 101 63 114 A1 und DE 197 53 665 A1 offenbart.

Der vorliegenden Erfindung liegt die Aufgabe zugrunde, ein verbessertes Tampon zur Verfügung zu stellen, das insbesondere eine bessere Handhabung beim Entfernen des Tampons ermöglicht. Diese Aufgabe wird mit den Merkmalen der unabhängigen Ansprüche gelöst.

Die Erfindung geht dabei entsprechend einer ersten Ausführungsform von dem Grundgedanken aus, ein Tampon bereitzustellen, das einen länglichen Tamponkörper mit einem Einführende, einem Mittelabschnitt und einem dem Einführende gegenüberliegenden Rückholende sowie eine Rückholeinrichtung aufweist. Die Rückholeinrichtung weist ein mit dem Tamponkörper verbundenes, flexibles Zugelement, das sich vom Tamponkörper weg erstreckt, und einen Griffabschnitt auf. Der Griffabschnitt stellt bezüglich des Zugelements eine erhebliche Querschnittserweiterung bereit. Durch diese Konstruktion ist die Handhabung des Tampons, insbesondere die Entfernung des Tampons, erheblich vereinfacht.

Gemäß einer bevorzugten Ausführungsform ist der maximale Querschnitt des Griffabschnitts der Rückholeinrichtung mindestens das 3-fache, vorzugsweise mindestens das 5-fache, stärker bevorzugt mindestens das 10-fache des Querschnitts des Zugelements. Eine derartige Querschnittserweiterung lässt sich beispielsweise durch einen kugel-, zylinder-, scheiben-, stern-, ring-, rauten-, herz-, blumen-, zahlen-, buchstaben-, schleifen- und/oder tierförmig ausgebildeten Griffabschnitt erzielen. Ein derartiger Griffabschnitt kann beispielsweise auf dem flexiblen Zugelement durch Auffädeln angeordnet sein. Der Griffabschnitt selbst ist vorzugsweise nicht schlaufenförmig ausgebildet. Das Zugelement kann beispielsweise an seinem freien Endabschnitt, d.h. dem dem Rückholende des Tamponkörpers gegenüberliegenden Endabschnitt, verknotet sein. Ferner ist bevorzugt, den Griffabschnitt transparent, koloriert, glitzernd und/oder bunt auszubilden.

Das Zugelement kann zur sicheren Anbringung am Tamponkörper zumindest im Verbindungsbereich mit dem Tamponkörper eine bandartige, flache Querschnittsform aufweisen. Alternativ dazu oder in Kombination damit kann das Zugelement auch schnurförmig ausgebildet sein.

Gemäß einer weiteren bevorzugten Ausführungsform ist das Zugelement der Rückholeinrichtung aus einem im wesentlichen transparenten Material gebildet, insbesondere einem flachen, bandartigen Kunststoffmaterial. Die Rückholeinrichtung ist vorzugsweise schlaufenförmig ausgebildet. Gemäß einer anderen Ausführungsform ist die Rückholeinrichtung nicht schlaufenförmig ausgebildet.

Gemäß einer anderen Ausführungsform der Erfindung weist das Tampon einen länglichen Tamponkörper mit einem Einführende, einem Mittelabschnitt und einem dem Einführende gegenüberliegenden Rückholende sowie eine Rückholeinrichtung auf. Diese Rückholeinrichtung weist einen mit dem Tamponkörper verbundenes, im wesentlichen transparentes Zugelement auf, das sich vom Tamponkörper weg erstreckt.

Das Zugelement muss jedoch nicht vollständig aus einem transparenten Material gebildet sein, sondern kann lediglich in dem außerhalb des Tamponkörpers vorgesehenen Bereich transparent sein. Der im Tamponkörper zur Befestigung des Zugelements vorgesehene Abschnitt des Zugelements kann auch aus einem nicht transparenten Material gebildet sein.

Das Zugelement weist vorteilhafter Weise eine bandartige, flache Querschnittsform auf. Diese kann im Verbindungsbereich mit dem Tamponkörper eine sicherere Befestigung des Zugelements am Tamponkörper ermöglichen. Im Bereich außerhalb des Tamponkörpers kann durch die flache Querschnittsform des Zugelements eine angenehmere Handhabung erzielt werden, da das flache Zugelement bei Zugbelastungen eine bessere Druckverteilung an den Fingern der Benutzerin ermöglicht.

Auch bei dieser Ausführungsform kann die Rückholeinrichtung ferner einen Griffabschnitt aufweisen, der bezüglich des Zugelements eine erhebliche Querschnittserweiterung aufweist. Der maximale Querschnitt des Griffabschnitts kann beispielsweise das 3-fache, vorzugsweise mindestens das 5-fache, stärker bevorzugt mindestens das 10-fache des Querschnitts des Zugelements betragen. Ferner ist bevorzugt, dass das Zugelement schlaufenförmig ausgebildet ist.

Das erfindungsgemäße Tampon ist beim Entfernen deutlich besser und komfortabler handhabbar als herkömmliche Tampons. Darüber hinaus bietet das erfindungsgemäße Tampon erhebliche Gestaltungsspielräume hinsichtlich des Designs der Rückholeinrichtung. So kann die Rückholeinrichtung bzw. deren Zugelement und/oder Griffabschnitt beispielsweise transparent ausgebildet sein, um das Zugelement weniger sichtbar und auffallend zu gestalten. Alternativ dazu kann das Zugelement und/oder der Griffabschnitt der Rückholeinrichtung auch bewusst auffällig ausgebildet werden, beispielsweise durch die Form, das Material und/oder die Farbe des Zugelements und/oder des Griffabschnitts.

Die verschiedenen Designoptionen für die Rückholeinrichtung des erfindungsgemäßen Tampons bieten eine zielgruppenorientierte Auswahl von Tampons für Frauen verschiedenster Altersstufen und/oder Geschmacksvorstellungen.
Dadurch wird ein neuer, unverkrampfter Umgang mit dem Thema "Menstruation" ermöglicht. Insbesondere wird neben der verbesserten Handhabbarkeit durch den erfindungsgemäßen Tampon ein über Generationen hinweg tabuisiertes Thema positiv enttabuisiert. Was bislang häufig als peinlich empfunden wurde, kann durch den erfindungsgemäßen Tampon, beispielsweise bei Verwendung humorvoller, subjektbezogener oder anderweitiger zielgruppenorientierter Applikationen am Zugelement in Form des Griffabschnitts dazu beitragen, das Thema "Menstruation" zu enttabuisieren.

Dementsprechend bietet das erfindungsgemäße Tampon eine Kombination von Hygiene mit verbesserter Funktionalität und verbessertem Design, wodurch über die rein praktischen Vorzüge des erfindungsgemäßen Tampons ein neues Selbstverständnis im Umgang mit der Menstruation ermöglicht und gefördert wird. Dies wird insbesondere mit der erfindungsgemäßen Rückholeinrichtung, die deutlich sichtbar tragbar ist erreicht. Dies steht im Gegensatz zu dem bei herkömmlichen Tampons vorhandenen Zugbändchen, die ein lediglich notwendiges Mittel zum Entfernen des Tampons darstellen. Die Verwendung eines transparenten Zugelements dient ebenfalls der Enttabuisierung des Themas "Menstruation", da das Zugelement deutlich weniger als übliche Zugelemente in Erscheinung tritt.

Nachfolgend werden bevorzugte Ausführungsformen des erfindungsgemäßen Tampons unter Bezugnahme auf die Zeichnungen näher beschrieben. Es zeigen:

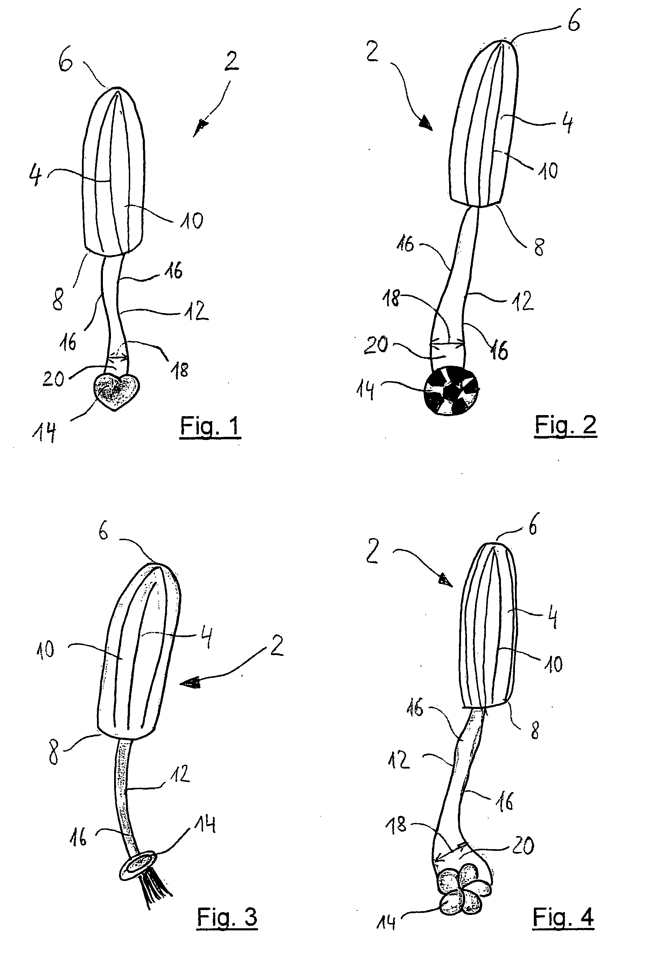

Figuren 1 bis 7 verschiedene Ausführungsformen des erfindungsgemäßen Tampons.

Zunächst wird auf die in Fig. 1 gezeigte Ausführungsform des erfindungsgemäßen Tampons 2 Bezug genommen. Das Tampon 2 weist einen länglichen Tamponkörper 4 mit einem Einführende 6, einem Mittelabschnitt und einem dem Einführende 6 gegenüberliegenden Rückholende 8 auf. Der Tamponkörper 4 kann mit Rillen oder Vertiefungen 10 versehen sein, die sich beispielsweise in Längsrichtung oder schraubenlinienförmig zumindest teilweise um den Umfang des Tamponkörpers 4 erstrecken.

Darüber hinaus weist das erfindungsgemäße Tampon 2 eine Rückholeinrichtung auf, die ein mit dem Tamponkörper 4 verbundenes, flexibles Zugelement 12 und einen Griffabschnitt 14 aufweist. Das flexible Zugelement kann aus jedem beliebigen körperverträglichen Material hergestellt sein, z.B. Baumwolle, Seide, Nylon, Vlies, Mikrofaser oder Samt. In der in Fig. 1 dargestellten Ausführungsform ist das Zugelement 12 schnurförmig, beispielsweise mit einem im wesentlichen runden Querschnitt, ausgebildet. Alternativ dazu kann das Zugelement 12 jedoch auch bandförmig, d.h. mit einer im wesentlichen flachen Querschnittsform, ausgebildet sein. Das Zugelement 12 ist vorzugsweise in einer beliebigen Farbe eingefärbt, kann jedoch auch transparent ausgebildet sein.

Der Griffabschnitt 14 kann jegliche Form aufweisen, die sicherstellt, dass der Griffabschnitt 14 bezüglich des Zugelements 12 eine erhebliche Querschnittserweiterung bereitstellt. Eine derartige erhebliche Querschnittserweiterung ist beispielsweise dann gegeben, wenn der maximale Querschnitt des Griffabschnitts 14 mindestens in einer Richtung, vorzugsweise senkrecht zur Längsrichtung des Zugelements, mindestens das 3-fache, vorzugsweise mindestens das 5-fache, stärker bevorzugt mindestens das 10-fache des Querschnitts des Zugelements 12 in derselben Richtung beträgt. Hat also beispielsweise das Zugelement 12 einen Durchmesser von 1 mm, so hat der Griffabschnitt 14 mindestens in einer Richtung, vorzugsweise in einer Richtung im wesentlichen senkrecht zur Längsrichtung des Zugelements 12, eine Abmessung von mindestens 3 mm. Eine derartige Querschnittserweiterung steht in klarem Gegensatz zu den bekannten Zugbändchen mit verknoteten Endabschnitt, bei denen der Querschnitt im Bereich des Knotens typischerweise höchstens etwa das 2-fache des Querschnitts des Zugelements beträgt.

Die Form des Griffabschnitts 14 ist in der in Fig. 1 gezeigten Ausführungsform des erfindungsgemäßen Tampons herzförmig. Es kommen jedoch verschiedenste andere Griffformen, z.B. Buchstaben, Zahlen, Glückssymbole (wie Hufeisen, Kleeblatt, Schweinchen, Münzen, Keep Smile, etc.), Tierkreiszeichen, Tiermotive, Wappen, Figuren (Nikolaus, Engel, Comicfiguren, etc.), Sportsymbole (Fußball, Tennisball, Golfball, Baseball, Boxhandschuhe, Rennwagen, Ballettschuhe, Ski, Segelschiff, etc.), Handschellen, Waffen, Sonne, Mond, Sterne, Blumenmotive, Pflanzen, Obst, Gemüse und ähnliches in Frage. Als Material für den Griffabschnitt 14 können sämtliche körperverträgliche Materialien, wie beispielsweise Baumwolle, Seide, Vlies, Mikrofaser, Kunststoff, Glas und ähnliches verwendet werden.

Die Anbringung des Griffabschnitts 14 am Zugelement 12 erfolgt vorzugsweise durch Auffädeln des Griffabschnitts 14 auf das Zugelement 12. Hierzu kann beispielsweise im Griffabschnitt 14 eine Durchgangsöffnung vorgesehen sein. Bei der Ausführungsform gemäß Fig. 1 ist der herzförmige Griffabschnitt 14 auf ein schlaufenförmig ausgebildetes Zugelement 12 aufgefädelt. Das Zugelement 12 erstreckt sich vorzugsweise in den Tamponkörper 4 hinein und ist darin gesichert. Dies erfolgt üblicherweise dadurch, dass das Zugelement 12 um die den Tamponkörper 4 bildende Materialbahn geschlungen wird, bevor die Materialbahn im Laufe des Herstellungsprozesses aufgerollt wird (siehe z.B. DE 197 53 665 A1). Die sich vom Tamponkörper 4 wegerstreckenden Enden des Zugelements 12 können beispielsweise nach dem Auffädeln des Griffabschnitts 14 miteinander verknotet werden. Gleichfalls ist es möglich, den Griffabschnitt 14 mit den jeweiligen Enden des Zugelements 12 direkt zu verknoten. Anstelle einer Verknotung kann auch jegliche andere bekannte Verbindungsart, z.B. Verkleben, Verschweißen oder ähnliches verwendet werden.

Bei der in Fig. 1 dargestellten Ausführungsform des erfindungsgemäßen Tampons 2 ist der Griffabschnitt 14 derart ausgebildet, dass die beiden das schlaufenförmige Zugelement bildenden Bänder 16 beabstandet voneinander gehalten werden. Dies ist in den Figuren durch den Pfeil 18 angedeutet. Diese gespreizte Anordnung der beiden Bänder 16 des Zugelements 12 ermöglichen ein leichteres Eingreifen in einen durch den Abstand 18 gebildeten Zwischenraum 20, was das Entfernen des Tampons 2 erheblich erleichtert.

Die erfindungsgemäße Rückholeinrichtung, insbesondere das Zugelement 12, kann aufgrund der besseren Greifbarkeit der Rückholeinrichtung durch den Griffabschnitt 14 und die vorzugsweise beabstandete Anordnung der Bänder 16 des Zugelements 12 kürzer ausgebildet werden als bei herkömmlichen Tampons. Dies hat zur Folge, dass ein mühseliges Suchen des Zugelements, wie dies bei herkömmlichen Tampons häufig erforderlich ist, überflüssig ist, da der Griffabschnitt 14 sofort greifbar ist.

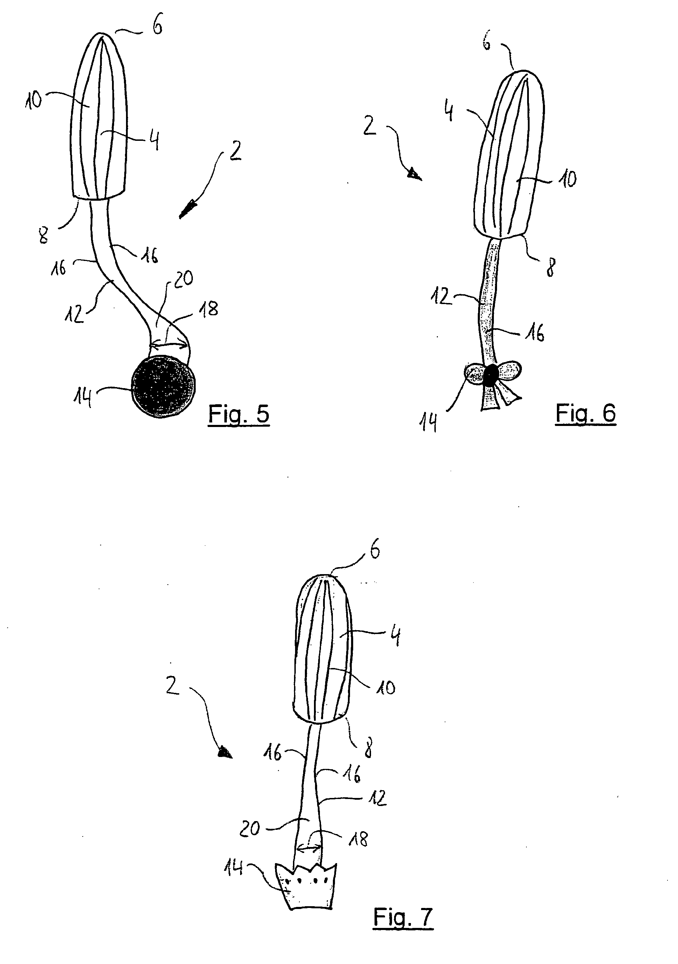

In den Figuren 2, 4, 5 und 7 sind weitere Ausführungsformen des erfindungsgemäßen Tampons 2 dargestellt, die im wesentlichen der in Fig. 1 dargestellten Ausführungsform entsprechen. Diese unterscheiden sich lediglich durch unterschiedliche Formen der Griffabschnitte 14. So ist beispielsweise in Fig. 2 ein fußballförmiger, in Fig. 4 ein blumenförmiger, in Fig. 5 ein kugelförmiger und in Fig. 7 ein kronenförmiger Griffabschnitt 14 vorgesehen.

Bei den in Figuren 3 und 6 dargestellten Ausführungsformen des erfindungsgemäßen Tampons ist die Rückholeinrichtung nicht schlaufenförmig, d.h. in Form eines einzigen Bandes 16 ausgebildet. Der Griffabschnitt 14 ist bei der in Fig. 3 dargestellten Ausführungsform durch einen Knoten ausgebildet. Bei der in Fig. 6 dargestellten Ausführungsform ist der Griffabschnitt 14 in Form einer Schleife ausgebildet.

Sämtliche Ausführungsformen des erfindungsgemäßen Tampons ermöglichen ein leichteres Greifen und Entfernen des Tampon, wobei gleichzeitig verschiedenste Designoptionen gegeben sind.

## Patentansprüche

1. Tampon, insbesondere für die Frauenhygiene, mit:
(a) einem länglichen Tamponkörper, der ein Einführende, einen Mittelabschnitt und ein dem Einführende gegenüberliegendes Rückholende aufweist, und
(b) einer Rückholeinrichtung, die ein mit dem Tamponkörper verbundenes, flexibles Zugelement, das sich vom Tamponkörper weg erstreckt, und einen Griffabschnitt aufweist, wobei der Griffabschnitt bezüglich des Zugelements eine erhebliche Querschnittserweiterung bereitstellt.

2. Tampon nach Anspruch 1, wobei der maximale Querschnitt des Griffabschnitts mindestens das 3-fache, vorzugsweise mindestens das 5-fache, stärker bevorzugt mindestens das 10-fache des Querschnitts des Zugelements ist.

3. Tampon nach Anspruch 1 oder 2, wobei der Griffabschnitt nicht schlaufenförmig ausgebildet ist.

4. Tampon nach einem der Ansprüche 1 bis 3, wobei der Griffabschnitt kugel-, zylinder-, scheiben-, stern-, ring-, rauten-, herz-, blumen-, zahlen-, buchstaben-, schleifen- und/oder tierförmig ausgebildet ist.

5. Tampon nach einem der Ansprüche 1 bis 4, wobei der Griffabschnitt koloriert, glitzernd und/oder bunt ausgebildet ist.

6. Tampon nach einem der Ansprüche 1 bis 5, wobei der Griffabschnitt auf das Zugelement aufgefädelt und daran gesichert ist.

7. Tampon nach einem der Ansprüche 1 bis 6, wobei das Zugelement aus einem im Wesentlichen transparenten Material gebildet ist.

8. Tampon, insbesondere für die Frauenhygiene, mit:
(a) einem länglichen Tamponkörper, der ein Einführende, einen Mittelabschnitt und ein dem Einführende gegenüberliegendes Rückholende aufweist, und
(b) einer Rückholeinrichtung, die ein mit dem Tamponkörper verbundenes, im Wesentlichen transparentes Zugelement aufweist, das sich vom Tamponkörper weg erstreckt.

9. Tampon nach einem der Ansprüche 1 bis 8, wobei das Zugelement zumindest im Verbindungsbereich mit dem Tamponkörper eine bandartige, flache Querschnittsform aufweist.

10. Tampon nach einem der Ansprüche 1 bis 9, wobei das Zugelement schnurförmig ausgebildet ist.

11. Tampon nach einem der Ansprüche 1 bis 10, wobei das Zugelement an seinem freien Endabschnitt verknotet ist.

12. Tampon nach einem der Ansprüche 1 bis 11, wobei die Rückholeinrichtung schlaufenförmig ausgebildet ist.

13. Tampon nach Anspruch 12, wobei der Griffabschnitt so ausgebildet ist, dass er die die Schlaufe bildenden Bänder der Rückholeinrichtung voneinander im Abstand anordnet.

14. Tampon nach einem der Ansprüche 1 bis 13, wobei die Rückholeinrichtung nicht schlaufenförmig ausgebildet ist.
